# Europäisches Patentamt
## European Patent Office
### Office européen des brevets

(19)

(11) Veröffentlichungsnummer: **0 083 417**
**B1**

(12) **EUROPÄISCHE PATENTSCHRIFT**

(45) Veröffentlichungstag der Patentschrift:
**12.09.84**

(51) Int. Cl.³: **C 07 C 79/46, C 07 C 76/02**

(21) Anmeldenummer: **82111074.9**

(22) Anmeldetag: **01.12.82**

(54) Verfahren zur Herstellung von 1-Nitro-benzol-2-carbonsäure-alkylester-5-carbonsäuren.

(30) Priorität: **08.12.81 DE 3148422**

(43) Veröffentlichungstag der Anmeldung:
**13.07.83 Patentblatt 83/28**

(45) Bekanntmachung des Hinweises auf die Patenterteilung:
**12.09.84 Patentblatt 84/37**

(84) Benannte Vertragsstaaten:
**CH DE FR GB LI**

(56) Entgegenhaltungen:
**EP - A - 0 033 829**

**Chemical Abstracts Band 76, Nr. 11, 13. März 1972, F. Muzlk et al. "Nitromonomethyl terephthalate", Abstract Nr. 59246g**

(73) Patentinhaber: **HOECHST AKTIENGESELLSCHAFT, Postfach 80 03 20, D-6230 Frankfurt am Main 80 (DE)**

(72) Erfinder: **Wykypiel, Werner, Dr., Egerstrasse 7, D-6054 Rodgau (DE)**
Erfinder: **Tronich, Wolfgang, Dr., Adolf-Guckes-Weg 5, D-6239 Eppstein/Taunus (DE)**

## Beschreibung

Die Erfindung betrifft eine Verfahrensverbesserung zur Herstellung von wertvollen Zwischenprodukten für Azoverbindungen, insbesondere Pigmente.

Aus der tschechischen Patentschrift Nr. 138287 ist ein Verfahren zur Herstellung von 1-Nitrobenzol-2-carbonsäuremethylester-5-carbonsäure bekannt. Bei diesem wird Benzol-1,4-dicarbonsäuredimethylester in rauchender Schwefelsäure mit einer Mischsäure aus 20%igem Oleum und 98%iger Salpetersäure nitriert; der erhaltene 1-Nitrobenzol-2,5-dicarbonsäuredimethylester wird sodann zunächst isoliert und anschliessend mit einem grossen Überschuss an 70%iger wässeriger Schwefelsäure partiell hydrolisiert. Die im sauren Medium abgeschiedene 1-Nitrobenzol-2-carbonsäuremethylester-5-carbonsäure wird sodann mittels Natriumcarbonat gelöst und mit Salzsäure wieder ausgefällt. Diesem Verfahren haftet bei einer technischen Durchführung der Nachteil einer sehr hohen Abwasserbelastung an. Ausserdem erfordert es eine zusätzliche Isolierung sowie eine Reinigungsoperation, und die Bildung von explosiblem Dimethyläther aus dem bei der Hydrolyse der Estergruppe entstehenden Methylalkohol ist nicht auszuschliessen.

Daraufhin wurde in der deutschen Offenlegungsschrift Nr. 3001695 ein verbessertes Verfahren der sauren Esterhydrolyse vorgeschlagen, bei welchem man zur Vermeidung von explosiblem Dimethyläther die Hydrolyse des 1-Nitrobenzol-2,5-dicarbonsäuredialkylesters mittels wässeriger Salpetersäure vornimmt. Allerdings hat auch dieses Verfahren im grosstechnischen Bereich gewisse Mängel, da bei ihm eine störende Bildung an nitrosen Gasen nicht ausgeschlossen werden kann.

Mit der vorliegenden Erfindung wurde nun ein verbessertes Verfahren zur Herstellung von 1-Nitrobenzol-2-carbonsäurealkylester-5-carbonsäure gefunden, mit dem die erwähnten Nachteile dieser bekannten Verfahren vermieden werden.

Die vorliegende Erfindung betrifft ein Verfahren zur Herstellung von 1-Nitrobenzol-2-carbonsäurealkylester-5-carbonsäuren, bei dem man von einem Benzol-1,4-dicarbonsäuredialkylester ausgeht, diesen nitriert und anschliessend der sauren Esterhydrolyse unterwirft, das dadurch gekennzeichnet ist, dass man die saure Esterhydrolyse ohne Zwischenisolierung des 1-Nitrobenzol-2,5-dicarbonsäuredialkylesters aus dem Nitrierungsansatz und in Gegenwart einer katalytischen Menge eines mit Wasser ganz oder teilweise mischbaren organischen Lösemittels mit einem Siedepunkt von oberhalb 90°C und/oder eines Emulgators durchführt.

Die Alkylgruppen in den Ausgangs-Diesterverbindungen bzw. in den Monoester-Endprodukten sind bevorzugt Alkylgruppen von 1 bis 4 C-Atomen, wie Äthoxygruppen und Methoxygruppen. Bevorzugt ist die Herstellung der 1-Nitrobenzol-2-carbonsäuremethylester-5-carbonsäure, wobei man entsprechend von dem Benzol-1,4-dicarbonsäuredimethylester ausgeht.

Die Nitrierung der Benzol-1,4-dicarbonsäuredialkylester-Verbindung kann in an und für sich bekannter Weise erfolgen, wie beispielsweise mit einer Schwefelsäure/Salpetersäure-Mischung oder mit 98 bis 100%iger Salpetersäure, vorzugsweise in Gegenwart von Schwefelsäure, bei einer Temperatur von etwa 20 bis 30°C. Die der Nitrierungsreaktion folgende partielle saure Esterhydrolyse wird im wässerigen Medium unter Zusatz der erwähnten katalytischen Menge an einem organischen Lösemittel und/oder einem Emulgator durchgeführt. Hierfür kann der Nitrierungsansatz nach Beendigung der Nitrierungsreaktion mit Wasser oder mit verdünnter Schwefelsäure verdünnt werden, vorzugsweise bis zu einem Schwefelsäuregehalt von etwa 60 bis 85 Gew.-%. Die saure Hydrolyse erfolgt mittels des Überschusses an dem Schwefelsäure/Salpetersäure-Gemisch.

Das als Katalysator verwendete organische Lösemittel bzw. der Emulgator wird in einer Menge von bis zu 6 Gew.-%, beispielsweise von 0,1 bis 5 Gew.-%, vorzugsweise von 0,2 bis 1 Gew.-%, berechnet auf die Benzol-1,4-dicarbonsäuredialkylester-Ausgangsverbindung, in den Reaktionsansatz eingesetzt.

Die saure Esterhydrolyse geschieht vorzugsweise bei einer Temperatur oberhalb 60°C, wie beispielsweise bei einer Temperatur zwischen 65 und 110°C, insbesondere zwischen 75 und 95°C.

Das erfindungsgemässe Verfahren kann beispielsweise in der Weise durchgeführt werden, dass man zunächst den Benzol-1,4-dicarbonsäuredialkylester in 93 bis 98%iger Schwefelsäure löst, mit einem Schwefelsäure/Salpetersäure- oder Oleum/Salpetersäure-Gemisch oder mit 98 bis 100%iger Salpetersäure versetzt und in diesem Medium bei einer Temperatur zwischen 15 und 35°C nitriert, den Ansatz anschliessend mit Wasser auf einen Säuregehalt von etwa 60 bis 85 Gew.-%, vorzugsweise 65 bis 75 Gew.-%, verdünnt, das organische Lösemittel oder den Emulgator oder beide zugibt und die saure Esterhydrolyse sodann bei einer Temperatur zwischen etwa 80 und 90°C ausführt. Nach Beendigung dieser Hydrolyse kühlt man den Ansatz auf etwa 20°C ab, verdünnt ihn mit kaltem Wasser und saugt die abgeschiedene 1-Nitrobenzol-2-carbonsäurealkylester-5-carbonsäure ab, wäscht sie mit Wasser und trocknet sie.

Organische, mit Wasser ganz oder teilweise mischbare Lösemittel, die erfindungsgemäss in katalytischer Menge in den Reaktionsansatz eingesetzt werden und die einen Siedepunkt von oberhalb 90°C besitzen sollen, sind beispielsweise Alkanole, wie n-Butanol, die Hexanole, Octanole, Cyclohexanol, die zwei- und dreiwertigen aliphatischen Alkohole und deren niederen Alkyläther, wie Äthylenglykol, Propylenglykol, Diäthylenglykol, Dipropylenglykol, Methyltrimethylenglykol, Hexamethylenglykol, Triäthylenglykol, Butandiol-1,4, Glycerin, Diglycerin, Äthylenglykolmonomethyläther, Äthylenglykolmonoäthyläther, Diäthylenglykolmonomethyläther, Diäthylenglykolmo-

noäthyläther, Diäthylenglykolmonopropyläther, Diäthylenglykolmonobutyläther, Triäthylenglykolmonopropyläther, Triäthylenglykolmonobutyläther, 3-Methoxybutanol, ebenso Thiodiäthylenglykol, γ-Chlorpropylenglykol, Benzylalkohol, Phenoxyäthanol, Chlorphenoxyäthanol, Furfurylalkohol, Tetrahydrofurfurylalkohol sowie cyclische aliphatische Äther, wie Dioxan oder Tetrahydrofuran, weiterhin Ester niederer Carbonsäuren mit niederen aliphatischen Alkoholen, wie Äthylenglykolacetat, Methylglykolacetat, Äthylacetat, Äthoxybutyrat, Glycerinacetat, niedere aliphatische Ketone, wie Aceton, Methyläthylketon, Methylisobutylketon oder Acetonylaceton, niedere aliphatische Monocarbonsäureamide von primären oder sekundären aliphatischen Aminen oder des Ammoniaks, wie Formamid, Dimethylformamid, Acetamid, Dimethylacetamid, N-Methylacetamid oder Bis-β-hydroxyäthylformamid, weiterhin mit Wasser mischbare aliphatische Hydroxyketone, wie Acetylcarbinol, Propionylcarbinol, Acetoin, Acetyldimethylcarbinol, 1-Hydroxy-3-acetylpropan, 1-Hydroxy-4-acetylbutan, Hydracetylaceton und besonders Diacetonalkohol, ebenso Nitrile niederer aliphatischer Carbonsäuren, wie Acetonitril, Äthylencyanhydrin, Propylencyanhydrin, Chlorcyanhydrin, Glykolsäurenitril, Milchsäurenitril, β-Hydroxypropionitril, Äthoxyacetonitril, β-Methoxy-propionitril, β-Äthoxypropionitril, β-Butoxypropionitril oder β-Hydroxy-β-äthoxypropionitril, desweiteren aliphatische Lactame, wie N-Methylpyrrolidon, aliphatische Lactone, wie Butyrolacton, Dialkylsulfoxide, wie Dimethylsulfoxid, Sulfolan, Sulfolen, Tetramethylensulfon, Bishydroxyäthylensulfon.

Emulgatoren, die erfindungsgemäss in katalytischen Mengen in den Reaktionsansatz eingesetzt werden, sind beispielsweise anionaktive Verbindungen mit Emulgatoreigenschaften, wie Salze von $C_8$-$C_{18}$-Fettsäuren mit Alkalimetallen, wie Natrium und Kalium, oder mit Metallen der 2. oder 3. Hauptgruppe, wie Calcium, Magnesium und Aluminium, von Ammoniak oder von aliphatischen Aminen, wie Morpholin, Triäthanolamin, Äthanolamin, Isopropanolamin, Salze von aliphatischen Schwefelsäureestern, wie Na-Laurylsulfat, Natriumsalze von Sulfobernsteinsäuredialkylestern, Schwefelsäureester von äthoxylierten Fettalkoholen und Alkylphenolen, Türkischrot, Salze von aromatischen Sulfonsäuren, wie Alkylbenzolsulfonate, Xylol- und Naphthalinsulfonate, Alkylnaphthalinsulfonate, ebenso Polyphosphate und Fettsäureanlagerungsprodukte mit hydrophilen Gruppen, z.B. Eiweisskondensationsprodukte, desweiteren kationaktive Verbindungen mit Emulgatoreigenschaften, wie beispielsweise Fettamine, quartäre Ammoniumverbindungen, wie Trimethylacetylammoniumchlorid, quartäre Verbindungen des Pyridins, des Morpholins und Imidazolins, wie Laurylpyridiniumchlorid, desweiteren ampholytische Verbindungen mit Emulgatoreigenschaften, wie langkettige substituierte Aminosäuren, beispielsweise N-Alkyldi(aminoäthyl)glycin, N-Alkyl-2-aminopropionat, Betaine, wie (3-Acylaminopropyl)dimethylglycin und Alkylimidazoliumbetain, desweiteren nichtionogene Verbindungen mit Emulgatoreigenschaften, wie partielle Ester von Polyalkoholen, beispielsweise Glyzerinmono- und -distereate und -oleate, Sorbitmonostearat und -oleat, ebenso Äthylenoxid- bzw. Propylenoxid-Addukte, beispielsweise an Fettsäuren, Fettalkohole oder Fettamine (wobei diese Produkte auch kationaktive Eigenschaften haben können) oder an partielle Fettsäureester mehrwertiger Alkohole, vorzugsweise des Glyzerins und des Sorbits, an Alkylphenole, Wasser (Polyalkylenglykole) sowie an Amide höherer Fettsäuren, wie beispielsweise Stearinsäureäthanolamid, ferner Eiweissstoffe mit Emulgatoreigenschaften und deren Abbauprodukte mit Emulgatoreigenschaften, wie Gelatine, Albumine, Sericin, Natriumnucleinnate, Zein und Kasein, weiterhin synthetische Hochpolymere mit Emulgatoreigenschaften, wie Polyvinylalkohol, Polyvinylpyrrolidon und Carboxyvinylpolymerisat.

Das erfindungsgemässe Verfahren gegenüber bekannten Verfahren hat verschiedene Vorteile, wie insbesondere die, dass eine Isolierung der als Zwischenverbindung dienenden Nitroverbindung aus dem Nitrierungsansatz entfällt, die Abwasserbelastung auf ein Minimum reduziert ist und die als Endprodukt erhaltene 1-Nitrobenzol-2-carbonsäurealkylester-5-carbonsäure in erheblich besserer Ausbeute und in einer Reinheit erhalten wird, die für eine Weiterverarbeitung dieser Verbindung zum Carbonsäurechlorid zur anschliessenden Umsetzung zu entsprechenden Carbonsäurearylamiden erforderlich und ausreichend ist (die aus diesen Amiden durch Reduktion erhältlichen 1-Amino-2-carbonsäurealkylester-5-carbonsäurearylamide dienen als Diazokomponenten zur Herstellung von wertvollen Azopigmenten; vgl. deutsche Patentschrift Nr. 1 263 202).

Die nachstehenden Beispiele dienen zur Erläuterung der Erfindung. Teile sind Gewichtsteile und Prozentangaben sind Gewichtsprozente, sofern nicht anders vermerkt.

*Beispiel 1*

Zu einer Lösung von 250 Teilen Benzol-1,4-dicarbonsäuredimethylester in 420 Teilen 95%iger wässeriger Schwefelsäure gibt man unter Rühren unter schwacher Aussenkühlung bei einer Innentemperatur von 25 bis 30°C innerhalb von 2 h gleichmässig 297,5 Teile einer Mischung aus 208,2 Teilen 95%iger Schwefelsäure und 89,3 Teilen 100%iger Salpetersäure. Den Nitrierungsansatz rührt man anschliessend noch 4 h bei einer Temperatur von etwa 15 bis 25°C (Raumtemperatur) nach.

In dieses Reaktionsgemisch gibt man dann ohne weitere Aussenkühlung innerhalb von 15 Min stetig 223 Teile Wasser hinzu; hierbei steigt die Temperatur auf etwa 80°C an, und durch diese Massnahme wird eine Schwefelsäurekonzentration von etwa 70% eingestellt. Es werden 0,5 Teile Äthylenglykol hinzugegeben, und der Reaktionsansatz wird noch 4 h bei 80°C weitergerührt. Man kühlt sodann auf 20°C ab, rührt 1500 Teile Wasser ein, rührt 30 Min bei 20°C weiter, saugt das feinkörnig angefallene Produkt ab und wäscht es mit etwa 4000 Teilen Wasser säurefrei und trocknet es.

Man erhält 249 Teile 1-Nitrobenzol-2-carbonsäuremethylester-5-carbonsäure mit einem Reingehalt von 95%, was 236,5 Teilen eines 100%igen Produktes und somit einer Ausbeute an 81,6% der Theorie entspricht.

Zu dem selben Ergebnis gelangt man, wenn man das Nitriergemisch in ein zweites Gefäss mit Wasser drückt und nach Einstellung der erforderlichen Schwefelsäurekonzentration die Hydrolyse vornimmt.

*Vergleichsbeispiel*

Man verfährt in der im Beispiel 1 angegebenen Verfahrensweise, jedoch mit dem Unterschied, dass man die Verseifung der 1-Nitrobenzol-2,5-dicarbonsäuredimethylester-Verbindung ohne Äthylenglykol durchführt. Man erhält nur eine klumpige, nicht filtrierbare, unreine Masse.

*Beispiele 2 bis 14*

Man verfährt analog der Verfahrensweise des Beispieles 1, verwendet jedoch in erfindungsgemässer Weise anstelle des Äthylenglykols in äquivalenter Menge einen der in den nachstehenden Tabellenbeispielen angegebenen Emulgatoren oder eines der Lösemittel. Man erhält das Endprodukt in vergleichbar guter Reinheit und Ausbeute. Die Werte für die Ausbeuten, umgerechnet auf Ausbeuten in Prozent der Theorie, sind in den Tabellenbeispielen angegeben.

| Bsp. | erfindungsgemäss verwendeter Katalysator | Ausbeute (% d. Th.) |
|---|---|---|
| 2 | Umsetzungsprodukt von 19 Mol Äthylenoxid mit 1 Mol des Additionsproduktes von Camphan an p-Kresol | 87,6 |
| 3 | Kondensationsprodukt aus 1 Mol Orthophosphorsäure, aus 1 Mol eines Umsetzungsproduktes von 2 Mol Äthylenoxid mit 1 Mol Laurylalkohol und aus 1 Mol eines Polyglykols (mit durchschnittlichem Molekulargewicht von 300) | 87,1 |
| 4 | Dodecyltrimethylammoniumchlorid | 87,1 |
| 5 | Stearyldimethylbenzylammoniumchlorid | 82,6 |
| 6 | Cetylpyridiniumchlorid | 81,5 |
| 7 | Kondensationsprodukt aus Formaldehyd und Naphthalinsulfonsäure (Natriumsalz) | 79,6 |
| 8 | Polyglykol mit durchschnittlichem MG 200 | 82,5 |
| 9 | Polyglykol mit durchschnittlichem MG 600 | 81,5 |
| 10 | Triäthylenglykol | 84,8 |
| 11 | Dimethylformamid | 81,7 |
| 12 | Octylalkohol | 81,7 |
| 13 | Dimethylsulfoxid | 81,6 |
| 14 | Umsetzungsprodukt von 20 Mol Äthylenoxid mit 1 Mol Oleylalkohol | 82,1 |

## Patentansprüche

1. Verfahren zur Herstellung von 1-Nitrobenzol-2-carbonsäurealkylester-5-carbonsäuren durch Nitrierung von Benzol-1,4-dicarbonsäuredialkylestern und anschliessende partielle saure Esterhydrolyse des erhaltenen 1-Nitrobenzol-2,5-dicarbonsäuredialkylesters, dadurch gekennzeichnet, dass man die saure Esterhydrolyse ohne Zwischenisolierung des 1-Nitrobenzol-2,5-dicarbonsäuredialkylesters und in Gegenwart einer katalytischen Menge eines mit Wasser ganz oder teilweise mischbaren organischen Lösemittels mit einem Siedepunkt von oberhalb 90°C und/oder eines Emulgators durchführt.

2. Verfahren nach Anspruch 1, dadurch gekennzeichnet, dass man die Hydrolyse bei einer Temperatur zwischen 65 und 110°C durchführt.

3. Verfahren nach Anspruch 1, dadurch gekennzeichnet, dass man die Hydrolyse bei einer Temperatur zwischen 75 und 95°C durchführt.

## Claims

1. Process for the preparation of 1-nitrobentene-2-alkoxycarbonyl-5-carboxylic acid by nitrating dialkyl ester of benzene-1,4-dicarboxylic acid and subsequent partial acidic ester-hydrolysis of the dialkyl ester of 1-nitro-benzene-2,5-dicarboxylic acid obtained, characterized in that the acidic ester-hydrolysis is carried out without intermediate isolation of the dialkyl ester of the 1-nitro-benzene-2,5-dicarboxylic acid and in the presence of a catalytic amount of an organic solvent which is completely or partially miscible with water and has a boiling point of above 90°C, and/or in the presence of an emulsifier.

2. The process according to claim 1, characterized in that the hydrolysis is carried out at a temperature between 65 and 110°C.

3. The process according to claim 1, characterized in that the hydrolysis is carried out at a temperature between 75 and 95°C.

## Revendications

1. Procédé de préparation de nitro-1 carboxy-5 benzènecarboxylates d'alkyles par nitration d'esters dialkyliques de l'acide benzène-dicarboxylique-1,4, puis hydrolyse acide partielle du nitro-1 benzène-dicarboxylate-2,5 de dialkyle obtenu, procédé caractérisé en ce qu'on effectue l'hydrolyse acide de l'ester sans isoler intermédiairement l'ester dialkylique de l'acide nitro-1 benzène-dicarboxylique-2,5 et en présence d'une quantité catalytique d'un solvant organique totalement ou partiellement miscible à l'eau et bouillant au-dessus de 90°C et/ou d'un émulsionnant.

2. Procédé suivant la revendication 1, caractérisé en ce qu'on effectue l'hydrolyse à une température comprise entre 65 et 110°C.

3. Procédé selon la revendication 1, caractérisé en ce qu'on effectue l'hydrolyse à une température comprise entre 75 et 95°C.